# EUROPÄISCHE PATENTANMELDUNG

(11) **EP 0 759 473 A1**
(43) Veröffentlichungstag der Anmeldung: **26.02.1997**
(21) Anmeldenummer: 95113211.7
(22) Anmeldetag: 23.08.1995
(51) Int. Cl.: C12N 15/57, C12N 9/52, C12N 1/21, C12N 15/74

(54) **Deletiertes Lysostaphingen von Staphylococcus Simulans**

(71) Anmelder: Karl Müller GmbH & Co., D-70469 Stuttgart (DE)
(72) Erfinder: Hertel, Christian, D-71144 Steinenbronn (DE); Cavadini, Christoph, D-70599 Stuttgart (DE); Hammes, Walter, Prof. Dr., D-70794 Filderstadt (DE)
(74) Vertreter: Thiel, Christian, Dr. Dipl.-Chem.

(57) **Zusammenfassung**

Die Erfindung betrifft ein deletiertes Lysostaphingen von Staphylococcus simulans, das Deletionen im Bereich der Repeats des Pro-Abschnitts, vorzugsweise mit der Basensequenz CTGAAGTAG AGACTTCAAA AGCXCCYGTA GAAAATACA G, wobei X für T oder C steht und Y für A oder G, aufweist, dieses Gen enthaltende Plasmide und Mikroorganismen sowie die Verwendung dieser Mikroorganismen zur Erzeugung von Lebensmitteln.

## Beschreibung

Die Erfindung betrifft ein deletiertes Lysostaphingen von Staphylococcus simulans, das Deletionen im Bereich der Repeats des Pro-Abschnitts, vorzugsweise mit der Basensequenz CTGAAGTAG AGACTTCAAA AGTXCCYGTA GAAAATACA G, wobei X für T oder C steht und Y für A oder G, aufweist, Plasmide, die dieses deletierte Lysostaphingen enthalten sowie Mikroorganismen, insbesondere der Gattung Lactobacillus und der Art Lactobacillus curvatus oder sake, die dieses deletierte Lysostaphingen in expressionsfähiger Form enthalten.

Erfindungsgemäße Lactobazillen der Arten L. curvatus und L. sake wurden nach den Bestimmungen des Budapester Vertrages bei der DSM unter den Bezeichnungen L. curvatus LTH 3985, 3986 und 3987 sowie L. sake LTH 4007 hinterlegt (DSM 9288, 9289, 9290, 9291).

Bakterien und insbesondere Milchsäurebakterien werden seit langem als Starterkulturen bei der Lebensmittelfermentierung eingesetzt. Nur beispielsweise sei auf die Fermentierung von rohen Pökelfleischwaren (Absäuerung) und die Verarbeitung von Milchprodukten hingewiesen. Sie tragen wesentlich dazu bei, hygienisch sichere und lagerfähige Produkte mit erwünschten sensorischen Eigenschaften zu erzeugen. Der konsevierende Effekt der Fermentierung mit Milchsäurebakterien beruht im wesentlichen auf der Bildung von Säuren u. a. Nebenprodukten, wie Wasserstoffperoxid, und teilweise auf der Erzeugung von spezifisch antimikrobiell wirkenden Substanzen, wie Bacteriocinen.

DE-C-37 39 989 beschreibt den Mikroorganismus Lactobacillus curvatus DSM 4265, der zur Herstellung von Pökelfleischwaren geeignet ist. Die Reifung unter kontrollierten Klimabedingungen dauert etwa 10 Tage und führt zu einer Absenkung des pH-Werts, zur Ausbildung eines typischen Aromas sowie zu einer Stabilisierung der Rohwurst durch Behinderung der spontanen Säurebildnerflora.

Es hat sich gezeigt, daß der Reifungsprozeß mit solch herkömmlichen Lactobazillen bei relativ hohen Reifungstemperaturen von bis zu 25° C immer in Konkurrenz zur Spontanflora abläuft. Da der Reifungsprozeß relativ langwierig ist, nimmt die Keimzahl der Spontanflora nicht unerheblich zu, was unter hygienischen Gesichtspunkten wie unter dem Gesichtspunkt der Kontrollierbarkeit des Reifungsprozesses unerwünscht ist. Insbesondere unerwünscht ist die Gegenwart von Staphylococcus aurens, da dieser in Lebensmitteln Toxine zu bilden vermag. Dies kann bei deren Verzehr zu gesundheitlichen Beeinträchtigungen führen.

Zur Unterdrückung der unerwünschten Spontanflora wurden auch besonders wachstumsstarke Stämme der Gattung Lactobacillus entwickelt. Es hat sich aber gezeigt, daß diese wachstumsstarken und konkurrenzfähigen Stämme mit der Zeit zur Selektionierung einer konkurrenzfähigen Spontanflora führen können, die den Vorteil der Wachstumsstärke wieder zunichte machen. Außerdem ist es erforderlich, auch diese wachstumsstarken Stämme in relativ hohen Keimzahlen einzusetzen, damit sie sich gegenüber der Spontanflora durchsetzen können. Erst der hierdurch vermittelte Wachstumsvorsprung führt zur weitgehenden Unterdrückung der Spontanflora.

Es ist schließlich aus DE-A-43 28 368 bekannt, daß gewisse Stämme, insbesondere auch der Gattung Lactobacillus, sogenannte Bacteriocine freisetzen, die die Vermehrung anderer Stämme der gleichen Gattung und auch andere Gattungen unterdrücken. Bacteriocine sind Peptide, Proteine oder Proteinkomplexe mit bacteriocider Wirkung, die vor allem gegen artverwandte Mikroorganismen wirksam werden und deren Wachstum unterdrücken. Bacteriocinerzeugende Milchsäurebakterien, wie Lactobacillus curvatus DSM 8430 oder Lactobacillus sake DSM 6742 sind zwar geeignet, die Spontanflora artverwandter Milchsäurebakterien zu unterdrücken, jedoch wird diese Eigenschaft nur in einer relativ engen Bandbreite gegenüber einer geringen Anzahl von Milchsäurebakterien wirksam. Bakterien anderer Gattungen, wie beispielsweise Staphylokokken, werden hierdurch nicht beeinträchtigt.

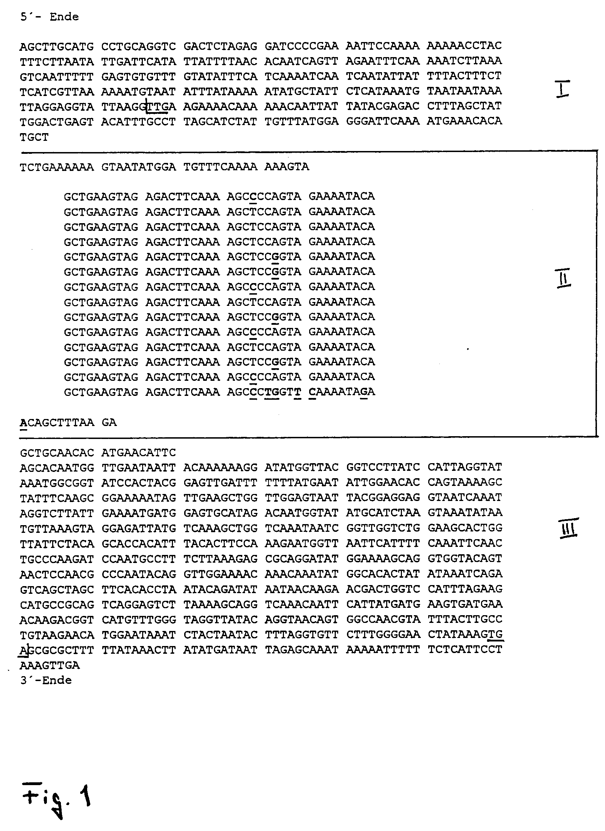

Es ist schließlich bekannt, daß Staphylococcus simulans ein auf andere Staphylokokken lysierend wirkendes Peptid erzeugen, das Lysostaphin. Das Lysostaphingen von Staphylococcus simulans wurde isoliert und sequenziert, P. Heinrich et al., Mol. Gen. Genet. 209, 563-569. Die hier verwendete Sequenz ist in Fig. 1 wiedergegeben, wobei es sich versteht, daß nur ein Strang der aus Basenpaaren bestehenden Sequenz dargestellt ist.

Die Möglichkeit, das für Lysostaphin kodierende Gen von Staphylococcus simulans zur Expression von Lysostaphin in anderen Mircoorganismen zu nutzen, wurde bereits diskutiert, wobei insbesondere auch die Expression in Lactobazillen angesprochen wurde. Es war zwar möglich, das vollständige Gen mit Hilfe von Plasmidvektoren in Mikroorganismen der Art Lactobacillus casei einzuschleusen (W. Gaier et al., Letters in Applied Microbiology 1992, 14, 72-76), jedoch war der transformierte Mikroorganismus nicht zur Expression von Lysostaphin in ausreichenden Mengen fähig und auch nicht mehr hinreichend vermehrungsfähig.

Das Lysostaphin besteht aus drei deutlich unterscheidbaren Abschnitten I bis III, von denen Abschnitt I den Promotor aufweist und ein Signalpeptid kodiert, Abschnitt II, der eine Abfolge von 14 Repeats der Basenfolge GCTGAAGTAG AGACTTCAAA AGCTCCAGTA GAAAATACA oder Varianten davon aufweist, für eine Prosequenz kodiert und Abschnitt III für das reife Lysostaphin. Anfang (TTG) und Ende (TGA) des Leserahmens sind in Fig. 1 markiert. Unterstreichungen im Abschnitt II zeigen Abweichungen in der Basenfolge an. Die vollständige Expression der Sequenzabschnitte I bis III erzeugt ein sogenanntes Präprolysostaphin mit einer Sequenz von 480 Aminosäureresten, von denen 36 Aminosäurereste das Signalpeptid darstellen. Nach Abspaltung des Signalpeptids bei Durchgang des Präpolysostaphins durch die Zellmembran entsteht Prolysostaphin mit 444 Aminosäureresten, das durch extrazelluläre Prozessierung weitere 198 Aminosäurereste zum reifen Lysostaphin mit 246 Aminosäureresten verliert. Die extrazelluläre Prozessierung erfolgt durch eine Protease, die von dem Lysostaphin produzierenden Stamm von Staphylococcus simulans selbst erzeugt wird. Prolysostaphin selbst wirkt nur in geringem Ausmaß lysierend auf Staphylokokken, erst die Abspaltung des Pro-Teils zum reifen Lysostaphin läßt die lysierende Wirkung voll wirksam werden.

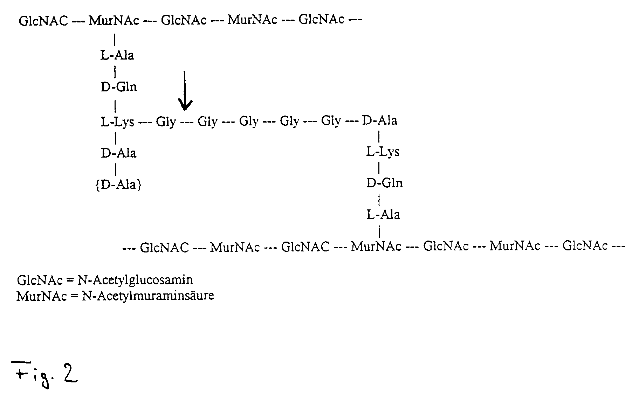

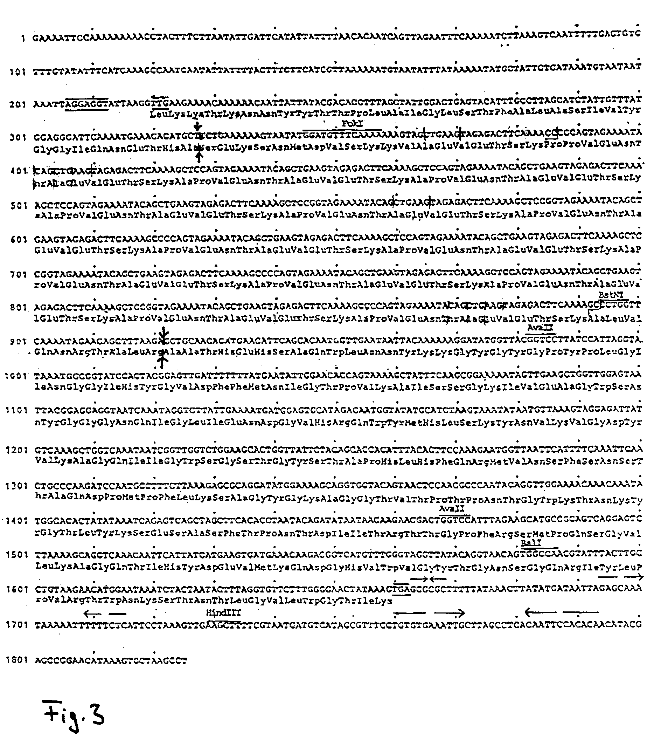

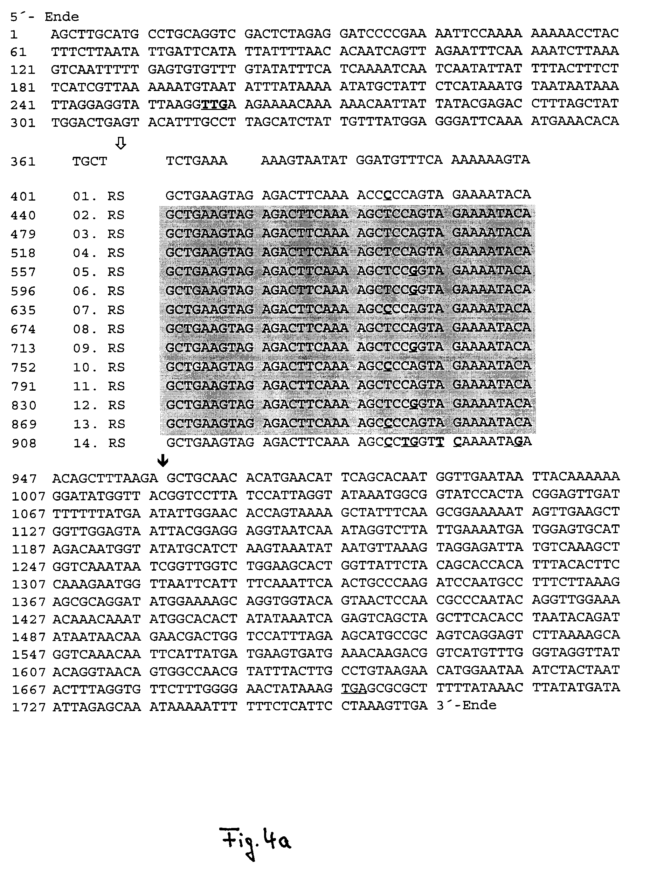

Das von Staphylococcus simulans gebildete Lysostaphin wirkt auf in der Hülle von nahezu allen Staphylokokken vorhandene Pentaglycin-Peptidbrücken lysierend. Fig. 2 zeigt einen Ausschnitt aus der Hüllstruktur von Staphylokokken mit der Pentaglycin-Brücke, die an der mit Pfeil bezeichneten Stelle von der Glycylglycin-Endopeptidase Lysostaphin angegriffen wird. Fig. 3 zeigt die Peptidsequenz von Lysostaphin und seiner Vorläufer (Spaltstellen sind durch Pfeile markiert).

Aufgrund der besonderen Eigenschaften von Lysostaphin insbesondere auch gegenüber lebensmitteltechnisch unerwünschten Staphylokokken der Art S. aureus wäre es wünschenswert, bei der Lebensmittelfermentierung verwandte Mikroorganismen, insbesondere auch der Gattung Lactobacillus, mit dem Lysostaphingen auszustatten, um unerwünschtes Wachstum von Staphylokokken durch in situ Produktion von Lysostaphin zu verhindern. In diesem Zusammenhang treten aber eine Reihe von Problemen auf, die sich aus der Struktur des Lysostaphingens wie auch aus der Struktur des von diesem Gen exprimierten Peptids ergeben.

So sind die Gründe für die reduzierte Vermehrungsfähigkeit und geringe Expression des transformierten Mikroorganismus Lactobacillus casei, wie oben erwähnt, nicht bekannt. Des weiteren ist nicht bekannt, ob ein in Lactobacillen eingebrachtes Lysostaphingen, sofern es exprimiert werden kann, überhaupt zur Bildung reifen Lysostaphins führt. Die für die Abspaltung der Pro-Sequenz erforderliche Protease sollten in Lactobazillen nicht verfügbar sein.

Angesichts dessen liegt der Erfindung das Problem zugrunde, das Lysostaphingen so zu modifizieren, daß es zur Expression eines zumindest auf Staphylokokken lysierend wirkenden Peptids in Wirtszellinien, insbesondere in für die Fermentierung von Lebensmittel geeigneten Mikroorganismen, in der Lage ist. Des weiteren ist Aufgabe der Erfindung die Bereitstellung eines Vektors, mit dem ein derartig modifiziertes Lysostaphingen in Wirtszellinien, insbesondere für die Lebensmittelerzeugung geeignete Mikroorganismen beispielsweise von der Gattung Lactobacillus, eingeschleust werden kann.

Diese Aufgabe wird mit einem deletierten Lysostaphingen von Staphylococcus simulans gelöst, das Deletionen im Bereich der Repeats des Pro-Abschnitts II, vorzugsweise mit der Basensequenz CTGAAGTAG AGACTTCAAA AGCXCCYGTA GAAAATACA G, wobei X für T oder C steht und Y für A oder G, aufweist.

Es hat sich überraschend gezeigt, daß ein deletiertes Lysostaphingen, wenn mit Hilfe eines Vektors in Mikroorganismen der Gattung Lactobazillus eingebracht, zur Expression des entsprechenden Peptids in der Lage ist. Ein solches Peptid besteht aus der unveränderten PräSequenz, einer verkürzten Pro-Sequenz und dem reifen Lysostaphin, wobei sich gezeigt hat, daß das Expressionsprodukt das Signalpeptid auf erwünschte Weise abspaltet. Überraschend kommt es auch zur Abspaltung der verbliebenen Pro-Sequenz, obwohl der Lactobazillus nicht über die die Prosequenz abspaltende Protease von Staphylococcus simulans verfügen sollte. Das reife Lysostaphin entspricht dem natürlich von S. simulans erzeugten und ist voll wirksam gegenüber Staphylococcus aurens.

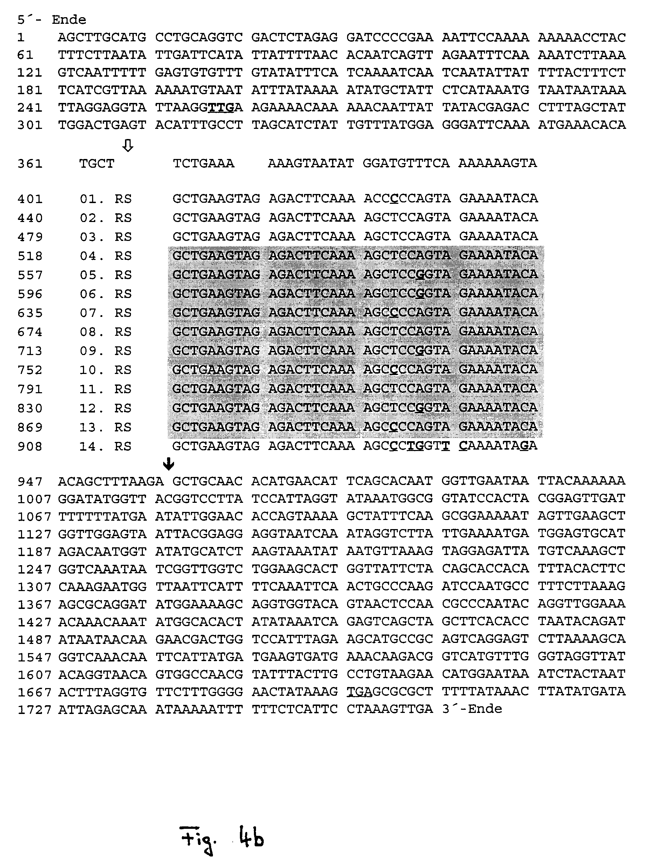

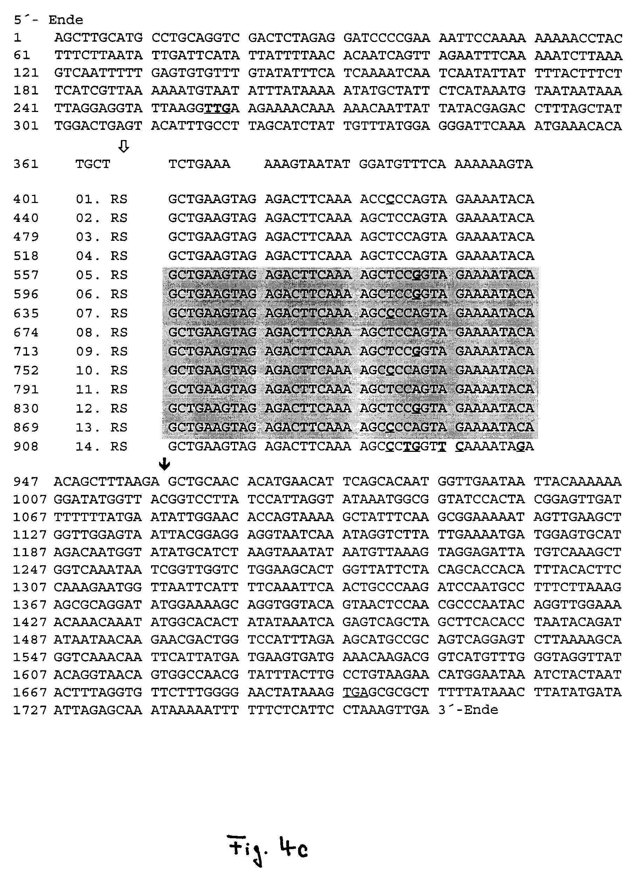

Im erfindungsgemäßen Lysostaphingen ist vorzugsweise wenigstens ein vollständiges Repeat im Abschnitt II, der für den Pro-Teil kodiert, deletiert. Als besonders vorteilhaft haben sich Deletionsprodukte erwiesen, in denen die Repeats 2 bis 13, 4 bis 13 oder 5 bis 13, wie aus Fig. 4a, b und c (eingerahmte Teile) ersichtlich, deletiert sind. Dabei beruht das Deletionsprodukt, in denen die Repeats 2 bis 13 deletiert sind, auf einer spontan aufgetretenen Deletion, das Deletionsprodukt, bei dem die Repeats 5 bis 13 bzw. 6 bis 13 deletiert sind, auf einem unvollständigen Verdau mit dem PvuII-Restriktionsenzym, das die am Übergang der Repeats 1 bis 13 zum darauffolgenden befindliche Basensequenz CAG/CTG erkennt und an mit bezeichneter Stelle schneidet. Dabei ist der PvuII-Verdau ohne weiteres steuerbar, so daß beliebige Repeats aus der Basensequenz herausgeschnitten werden können.

Es versteht sich, daß der Begriff "Deletion", wie hier verwandt, die Eliminierung der Repeats oder eines Teils davon des Lysostaphingens im Abschnitt II unter anschließender Ligation der großen Fragmente mit den Abschnitten I und III bezeichnet, wobei die Abschnitte I und III noch den Anfang bzw. das Ende des Abschnitts II einschließen können.

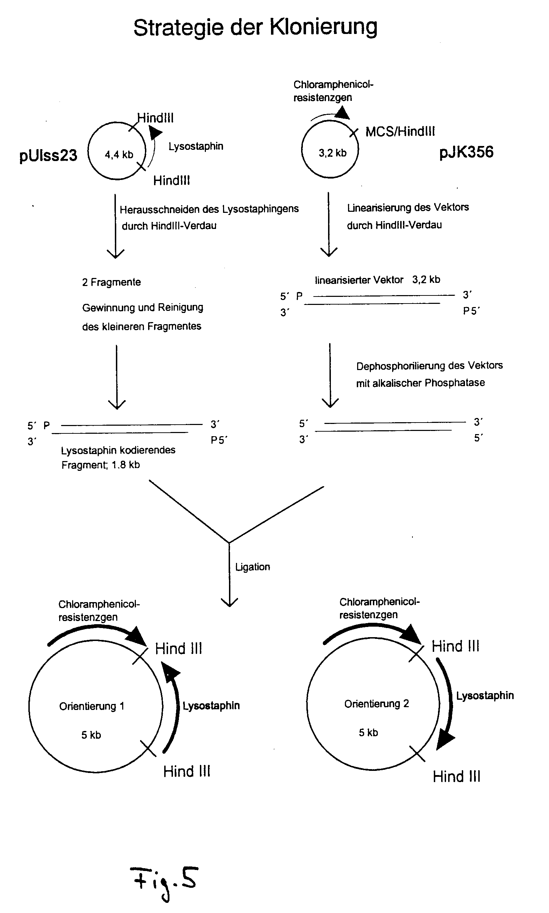

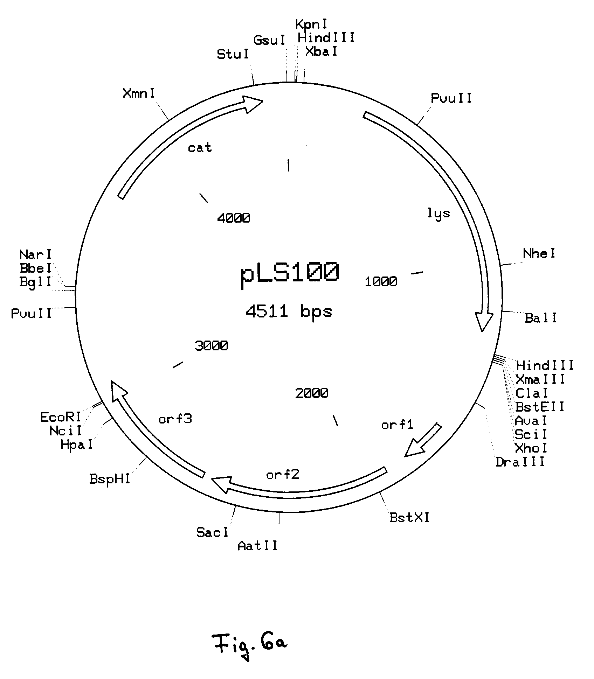

Die Erfindung betrifft des weiteren Plasmide, die das deletierte Lysostaphingen gemäß der Erfindung enthalten. Als besonders geeignet zur Transformation mit dem deletierten Lysostaphingen hat sich der Vektor pJK356 erwiesen (J. R. Klein, C. Ulrich und R. Plapp, Plasmid 30, 14-29 (1993)), der nach Ligation mit einem das Lysostaphingen enthaltenden Fragment die erfindungsgemäßen Plasmide pLS100, pLS200 und pLS201 ergab (Fig. 5).

Die erfindungsgemäßen Plasmide pLS100, 200 und 201 wurden nach Standardtechniken in Lactobacillen vom Stamm Lactobacillus curvatus LTH1432, hier auch als Lc2c bezeichnet, transformiert. Es zeigte sich überraschend, daß die so transformierten Stämme LTH1432(pLS200), LTH1432(pLS100) und LTH1432(pLS201) zur Expression des Gens in der Lage waren: Aktives Lysostaphin konnte in gewünschten Mengen im Kulturmedium nachgewiesen werden. Zudem zeigten die Stämme ein normales Wachstumsverhalten. Der mit dem vollständigen Lysostaphingen transformierte Stamm LTH/432 war zur Expression nicht in der Lage.

Anhand mehrerer Deletionsvarianten mit unterschiedlich langem Pro-Segment des Gens konnte gezeigt werden, daß die Expression des Gens durch die Deletion eines mehr oder weniger langen Teils des Pro-Segments gefördert wird, wobei die Länge der Deletion nicht von entscheidender Bedeutung ist. Offensichtlich ist in den unterschiedlichen Deletionsprodukten die Verkürzung des Pro-Teils des Peptids entscheidend für die Aufhebung der Inaktivierung des eigentlichen Lysostaphins.

Neben Lactobazillen der Art Lactobacillus curvatus konnten auch solche der Art Lactobacillus sake transformiert werden.

Die Erfindung betrifft schließlich die Verwendung von derart transformierten Mikroorganismen, inbesondere solchen der Gattung Lactobacillus, bei der Lebensmittelfermentierung. Besonders bevorzugt sind als Mikroorganismen Lactobacillus curvatus LTH1432(pLS200), LTH1432(pLS201), LTH1432(pLS100) und Lactobacillus sake LTH673(pLS200), die unter den Stammnummern LTH 3986, LTH 3987, LTH 3985 und LTH 4007 bei der DSM hinterlegt wurden.

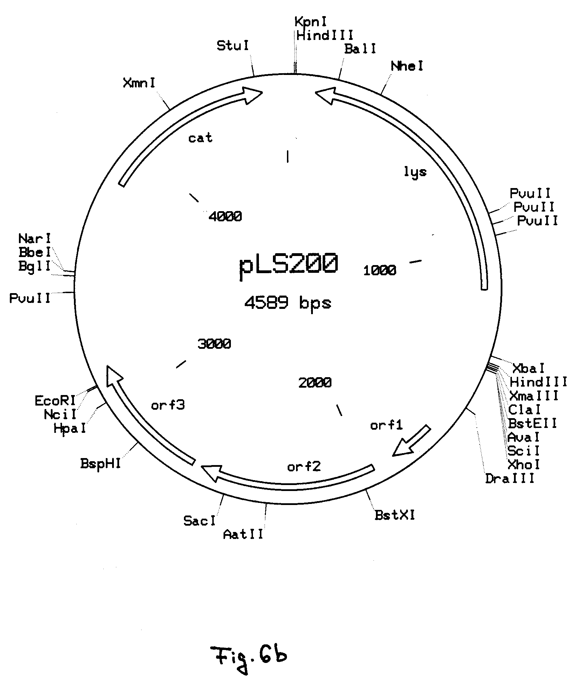

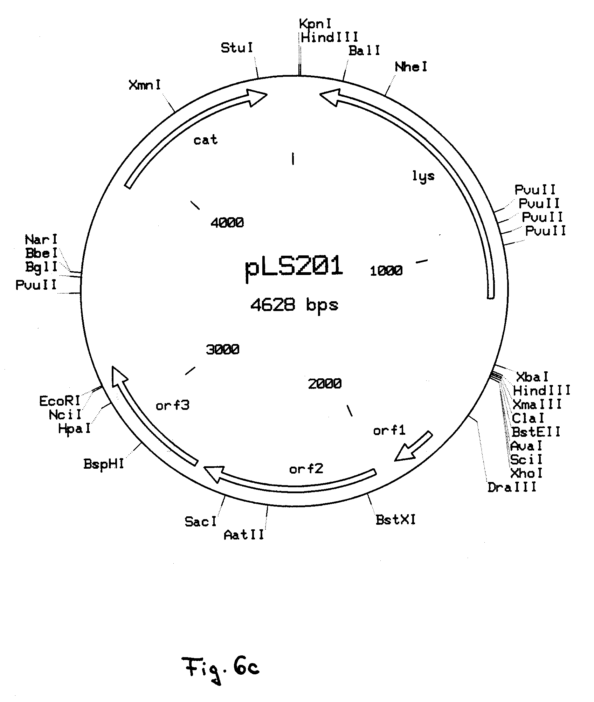

Das für die erfindungsgemäßen Modifizierungen verwandte Lysostaphingen von Staphylococcus simulans stand als HindIII-Fragment auf dem Plasmid pUlss23 zur Verfügung (P. Heinrich et al., Mol. Gen. Genet. 209, 563-569 (1987)). Das Gen wurde in den Vektor pJK356 (J. R. Klein et al., Plasmid 30, 14-29 (1993)) eingeführt, wobei drei verschiedene Plasmide pLS100, pLS200 und pLS201 erhalten wurden. pLS100 erwies sich als Plasmid mit spontan während der Konstruktion in Bacillus subtilis aufgetretener Deletion der Repeats 2 bis 13 (s. Fig. 3a). Die Plasmide pLS200 und pLS201 resultierten aus einem PvuII-Verdau des gesamten Vektors und anschließender Ligation der zwei großen Fragmente nach Aufreinigung mittels Agarosegel nach beschriebenen Techniken. Die Restriktionskarten von pLS100, pLS200 und pLS201 sind in Fig. 6a, b und c dargestellt, die Klonierungsstrategie zu ihrer Herstellung in Fig. 5.

Nach Protoplastentransformation in B. subtilis wurden Transformanten gefunden, die aktives Lysostaphin bilden. Die Plasmidanalyse der Transformanten ergab, daß alle kleinere Plasmide enthielten.

Die Plasmidisolierung aus B. subtilis und Transformation in Lactobacillus curvatus LTH1432 ergab Transformanten, die alle aktives Lysostaphin bildeten. Zwei Transformanten, die Plasmide unterschiedlicher Größe enthielten und Lysostaphin sekretierten, wurden zur Sequenzierung herangezogen und ergaben die in Fig. 4b und c dargestellten Sequenzen mit den darin eingerahmten Deletionen im Bereich der Repeats 1 bis 14.

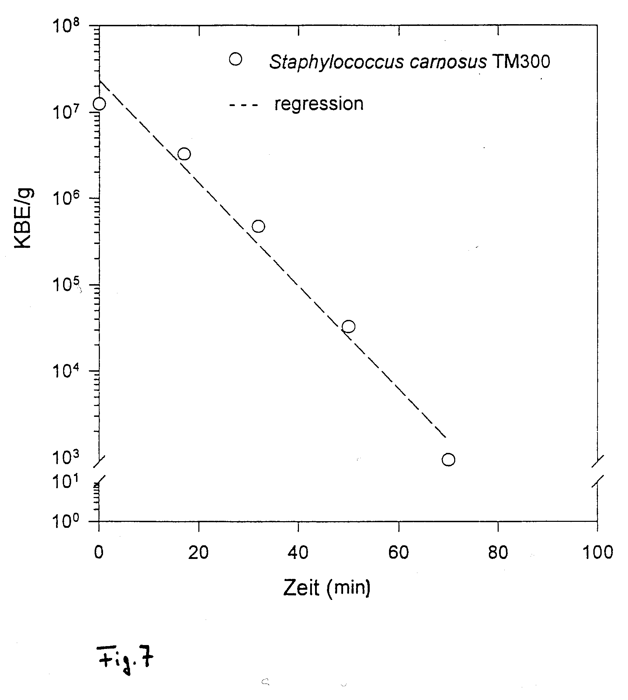

Für praktische Untersuchungen wurden die beiden oben erwähnten Stämme Lactobacillus curvatus LTH1432 (pLS200) und (pLS100) herangezogen. Die Behandlung von Staphylococcus carnosus TM300 mit dem lysostaphinhaltigen Überstand einer Übernachtkultur von L. curvatus LTH1432(pLS100) ergab eine logarithmische Inaktivierung der Indikatorzellen während des Wachstums der transformierten Lactobacillen (Fig. 7). Die Zahl der lebenden Staphylokokken konnte binnen einer Stunde um mehr als drei Größenordnungen herabgesetzt werden.

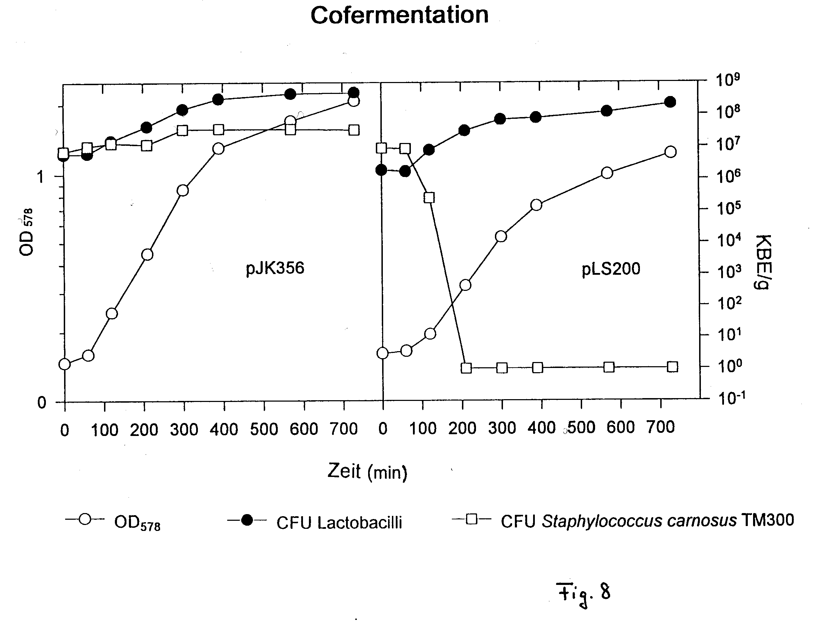

Weitere Konkurrenzversuche wurden mit Lactobacillus curvatus LTH1432 (pLS200) und Staphylococcus carnosus TM300 in einem üblichen Fermentierungsmedium vorgenommen. Zu Vergleichszwecken wurde der Versuch auch mit Lactobacillus curvatus LTH1432 (pJK356) vorgenommen, der den gleichen Ausgangsvektor, jedoch ohne das Lysostaphingen, enthielt. Wie in Fig. 8 dargestellt, ergab der Lysostaphin produzierende Stamm pLS200 bei Inokulation von jeweils etwa 10⁷ lebenden Zellen einen Rückgang der Staphylokokken auf praktisch 0 innerhalb von etwa 3 Stunden (Rechtecke), während die Lactobazillen auf einen Wert von etwa 5 x 10⁸ lebende Zellen pro ml zunahmen (schwarze Kreise). Im Vergleich mit dem nicht Lysostaphin produzierenden Lactobacillenstamm pJK356 wuchs die Staphylokokkenkonzentration bis auf einen Wert von nahezu 10⁹ lebenden Zellen pro ml an und nahmen damit stärker zu als die nicht Lysostaphin produzierenden Lactobacillen.

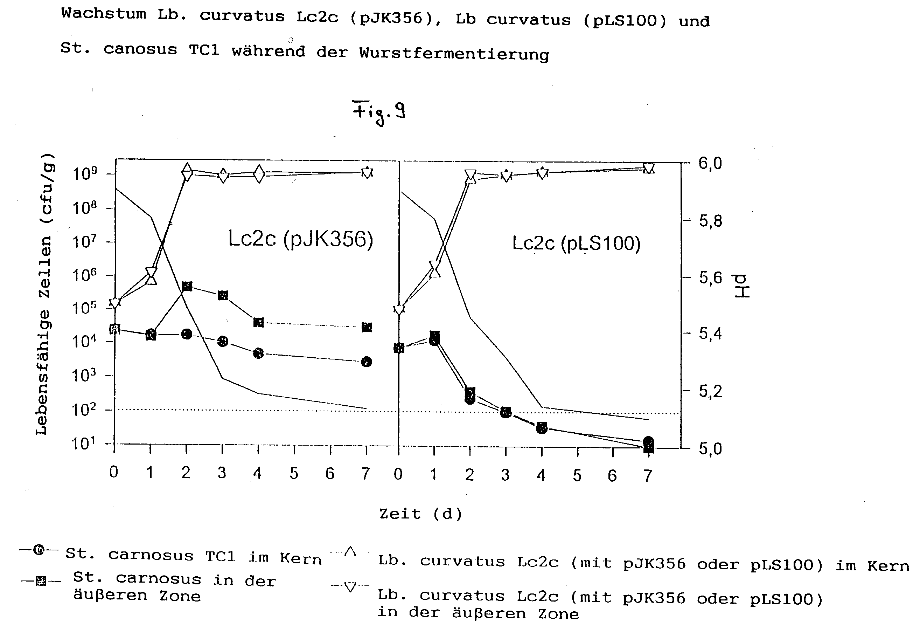

Weitere Versuche wurden bei der Rohwurstfermentierung (Salami) vorgenommen. Übliche Rohwurstmasse wurde mit 10⁵ koloniebildenden Zellen Lactobacillus curvatus LTH1432 und 10⁴ koloniebildenden Zellen pro g (cfu/g) Staphylococcus carnosus TC1 inoculiert, wobei einerseits der Lysostaphin produzierende Stamm LTH1432(pLS100) und andererseits als Kontrolle der nur den Vektor ohne Lysostaphingen enthaltende Stamm LTH1432(pJK356) verwandt wurden. Die Zahl der lebenden Zellen wurde in Abhängigkeit von der Zeit zusammen mit dem pH-Wert der Masse registriert, wobei hinsichtlich der Zellen noch zwischen der Kernzone und der Randzone der Wurstmasse unterschieden wurde, da sich erfahrungsgemäß Staphylokokken in der sauerstoffreichen Randzone besonders stark vermehren. Im Falle des aktiven Stammes LTH1432(pLS100) führte die Lysostaphinproduktion zu einer raschen Abnahme der Staphylokokken sowohl in der Kern- als auch in der Randzone im wesentlichen parallel zum Abfall des pH-Wertes. Im Falle des zur Kontrolle eingesetzten Stamms LTH1432(pJK356) nahm die Zahl der Staphylokokken in der Kernzone mit dem Abfall des pH-Wertes geringfügig ab, durchlief aber in der Randzone ein Maximum, um sich bei einem Wert oberhalb des Ausgangswertes zu stabilisieren. In beiden Fällen zeigten die Lactobazillen ein annährend gleiches Wachstum in Abhängigkeit von der Zeit. Die Ergebnisse sind in Fig. 9 wiedergegeben.

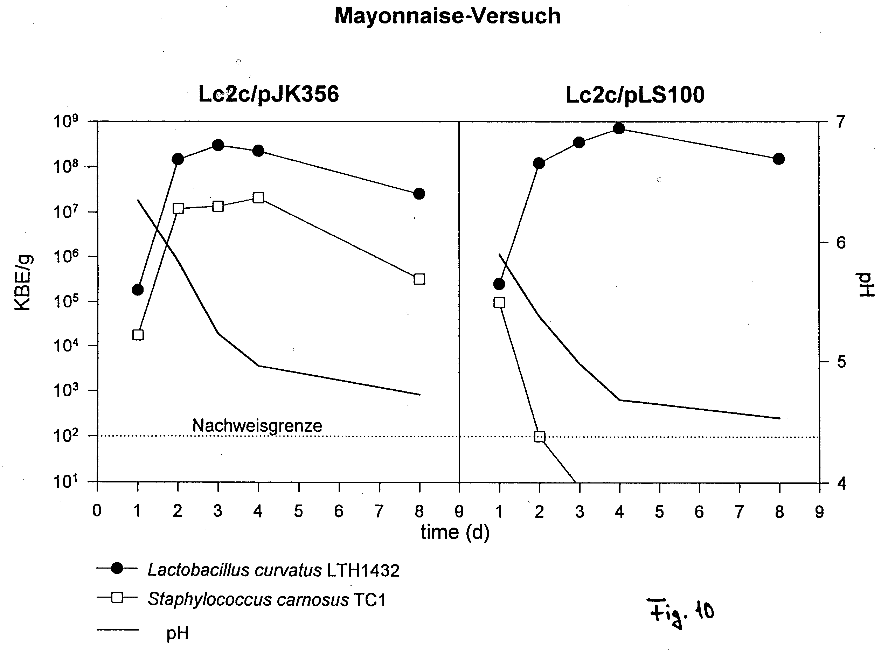

In einem weiteren Versuch wurde die Wirkung von Lysostaphin erzeugenden Lactobazillen auf Staphylokokken in Mayonnaise, wie sie in Feinkostsalaten verwandt wird, untersucht. Lactobacillus curvatus LTH1432 wurde zusammen mit Staphylococcus carnosus TC1 inoculiert und die Veränderung der Zahl der lebenden Zellen über die Zeit verfolgt. Einerseits wurde dabei der Lysostaphin erzeugende Stamm LTH1432(pLS100) verwandt, andererseits zur Kontrolle der nur den Vektor enthaltende Stamm L. curvatus LTH1432(pJK356). Mit dem Lysostaphin bildenden Stamm ergab sich eine Abnahme der Staphylokokken bis auf die Nachweisgrenze binnen 24 Stunden, während beim Kontrollversuch die Zahl der Staphylokokken erheblich zunahm und erst mit der Abnahme des pH-Wertes wieder geringfügig, auf einen Wert oberhalb des Inoculationswertes, abnahm. Die Ergebnisse sind in Fig. 10 zusammengefaßt.

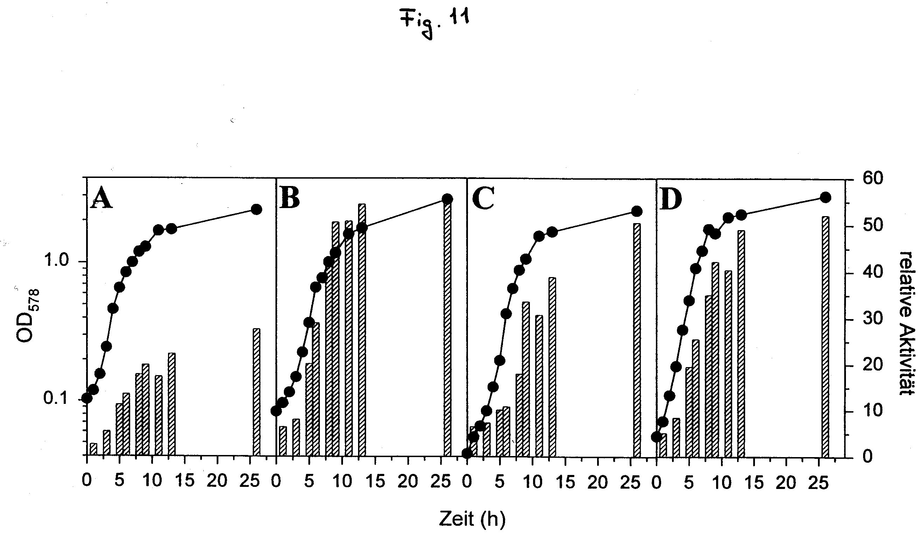

Fig. 11 zeigt schließlich den zeitlichen Ablauf des Kulturwachstums(0) von LTH/432 (pLS 100) (A), (pLS 200) (B), (pLS 201) (C) und LHT 673 (pLS 201) (D) sowie die relative staphylolytische Aktivität, berechnet als prozentuale Verminderung der optischen Dichte suspendierter Staphylokokkenzellen in 30 min.

Lysostaphin produzierende Mikroorganismen sind somit in der Lage, die Staphylokokkenzahl in Lebensmitteln zu kontrollieren. Dies macht ihre Verwendung bei der Lebensmittelfermentierung vorteilhaft. Zugleich können derartige Mikroorganismen aber auch als Schutzkultur in Fertiglebensmitteln eingesetzt werden, beispielsweise in Feinkostsalaten, die besonders anfällig für Staphylokokkenbefall sind. Eine geringfügige Säuerung bei Lactobacillen kann häufig in Kauf genommen werden oder ist erwünscht. Insbesondere kommt hier auch die Verwendung als Schutzkultur in Milchprodukten in Frage.

Nach bisherigen Erkenntnissen liegt das Lysostaphingen in den bisher damit transformierten Wirtsorganismen in Plasmidform vor. Insertierungen in den genetischen Strang des Wirtsorganismus wurden nicht beobachtet. Plasmidtransformierte Wirtszellen erweisen sich in nahezu allen Fällen als nicht stabil transformiert. In der Folge geht von Generation zu Generation mehr von dem in Plasmidform vorliegenden genetischen Material wieder verloren. Dies ist hier von Vorteil, da die transformierten Zellen bei der Lebensmittelfermentierung in lebensfähiger Form in die Umwelt gelangen, dort aber binnen kurzer Zeit die ihnen mit dem transformierten Plasmid beigebrachten Eigenschaften wieder verlieren.

## Patentansprüche

1. Deletiertes Lysostaphingen von Staphylococcus simulans, das Deletionen im Bereich der Repeats des Pro-Abschnitts aufweist.

2. Deletiertes Lysostaphingen nach Anspruch 1, dadurch gekennzeichnet, daß wenigstens eine vollständige Basensequenz CTGAAGTAG AGACTTCAAA AGCXCCYGTA GAAAATACA G, wobei X für T oder C steht und Y für A oder G, deletiert ist.

3. Deletiertes Lysostaphingen nach Anspruch 2, gekennzeichnet durch Deletionen der Repeats 2 bis 13, 4 bis 13 oder 5 bis 13.

4. Deletiertes Lysostaphingen nach einem der Ansprüche 1 bis 3, dadurch gekennzeichnet, daß die deletierte Basensequenz ein PvuII-Restriktionsfragment ist.

5. Plasmid, das ein deletiertes Lysostaphingen nach einem der Ansprüche 1 bis 4 enthält.

6. Plasmide pLS100, pLS200 und pLS201.

7. Mikroorganismus, dadurch gekennzeichnet, daß er das deletierte Lysostaphingen nach einem der Ansprüche 1 bis 4 enthält.

8. Mikroorganismus nach Anspruch 7, dadurch gekennzeichnet, daß er das deletierte Lysostaphingen in Form eines inkorporierten Plasmids enthält.

9. Mikroorganismus nach Anspruch 8, dadurch gekennzeichnet, daß er eines der Plasmide nach Anspruch 6 enthält.

10. Mikroorganismus nach Anspruch 7 bis 9 der Gattung Lactobacillus.

11. Mikroorganismus nach Anspruch 10 der Art Lactobacillus curvatus oder Lactobacillus sake.

12. Lactobacillus curvatus LTH 3985, 3986 und 3987 sowie Lactobacillus sake LTH 4007.

13. Verwendung der Mikroorganismen nach einem der Ansprüche 7 bis 12 zur Fermentierung von oder als Schutzkultur in Lebensmitteln.
